(19) Europäisches Patentamt
European Patent Office
Office européen des brevets

(11) Numéro de publication: 0 118 377 B1

# (12) FASCICULE DE BREVET EUROPEEN

(45) Date de publication du fascicule du brevet: **09.03.88**

(51) Int. Cl.⁴: **C 07 C 37/00**, C 07 C 39/27

(21) Numéro de dépôt: **84420029.5**

(22) Date de dépôt: **23.02.84**

(54) **Procédé de préparation de phénols métachlorés.**

(30) Priorité: **02.03.83 FR 8303648**

(43) Date de publication de la demande: **12.09.84 Bulletin 84/37**

(45) Mention de la délivrance du brevet: **09.03.88 Bulletin 88/10**

(84) Etats contractants désignés: **BE DE FR GB IT LU NL**

(56) Documents cités: **FR - A - 2 284 583**

(73) Titulaire: **RHONE-POULENC CHIMIE, 25, quai Paul Doumer, F-92408 Courbevoie Cédex (FR)**

(72) Inventeur: **Cordier, Georges, 50, Chemin des Hermières, F-69340-Francheville (FR)**

(74) Mandataire: **Vignally, Noel et al, RHONE-POULENC INTERSERVICES Service Brevets Chimie Centre de Recherches de Saint-Fons B.P. 62, F-69192 Saint-Fons Cedex (FR)**

EP 0 118 377 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un procédé de préparation de phénols comportant un atome de chlore sur au moins l'une des positions méta par rapport à l'hydroxyle phénolique, par déchloration de phénols plus fortement chlorés.

Par la suite on désignera par commodité les phénols comportant un atome de chlore sur au moins une des positions méta par «phénols métachlorés».

Les phénols métachlorés, et en particulier le chloro-3-phénol et le dichloro-3,5 phénol, sont des composés qui présentent un grand intérêt industriel comme intermédiaires en synthèse organique.

Diverses méthodes de préparation de phénols métachlorés ont été proposées. Elles comprennent notamment:

– des méthodes engendrant la fonction phénolique sur des composés aromatiques chlorés, telles que par exemple l'hydrolyse alcaline des polychlorobenzènes;

– des méthodes de chloration de phénols;

– des méthodes de déchloration de polychlorophénols.

Ces dernières méthodes présentent un intérêt industriel tout particulier en raison de la disponibilité des polychlorophénols dont certains sont des produits courants et d'autres des sous-produits d'intérêt limité, qu'il importe de valoriser.

C'est ainsi, par exemple, que l'on obtient des tri- et tétrachlorophénols isomères dont certains comportent un ou deux atomes de chlore en position méta par rapport à l'hydroxyle phénolique lors de la préparation du tétrachloro-2,3,4,6 phénol et du pentachlorophénol par chloration du dichloro-2,6 phénol, sous-produit de la préparation du dichloro-2,4-phénol. Ces divers polychlorophénols constituent des matières premières de choix pour la préparation des phénols méthachlorés par déchloration. Une méthode d'élimination des atomes de chlore excédentaires consiste à soumettre les polychlorophénols à une hydrogénation en phase vapeur ou en phase liquide en présence d'un catalyseur.

Certains procédés d'hydrodéchloration des polychlorophénols par l'hydrogène en phase liquide et en présence d'un catalyseur conduisent sélectivement à l'obtention des chloro-3 ou dichloro-3,5 phénols.

Ainsi la demande de brevet français n° 80/27 936 (publiée sous le n° 2 496 639) décrit un procédé de préparation de phénols métachlorés par déchloration sélective de polychlorophénols par l'hydrogène, en phase aqueuse acide comportant des ions halogénures et en présence d'un catalyseur à base d'un métal noble.

La demande de brevet français n° 80/27 937 (publiée sous le n° 2 496 640) a trait à un procédé voisin du précédent, dans lequel l'hydrodéchloration est conduite en phase aqueuse acide contenant des métaux lourds appartenant aux groupes 1b, 2b, 3a, 4a et 5a de la classification périodique des éléments et en présence d'un catalyseur à base d'un métal noble.

Enfin la demande de brevet français n° 80/27 938 (publiée sous le n° 2 496 641) décrit un procédé d'hydrodéchloration par l'hydrogène, en phase liquide organique et en présence d'un acide de Lewis et d'un catalyseur à base d'un métal noble.

Ces divers procédés sélectifs et économiquement intéressants ont comme point commun l'utilisation d'hydrogène en présence d'un catalyseur à base d'un métal noble pour réaliser la déchloration des polychlorophénols.

Il a maintenant été trouvé un procédé d'hydrodéchloration des polychlorophénols en phénols métachlorés par action de l'acide iodhydrique en phase liquide, qui s'affranchit de la nécessité d'utiliser un catalyseur et permet d'opérer à des températures moins élevées que dans l'art antérieur.

Plus précisément la présente invention concerne un procédé de préparation sélective de chlorophénol comportant un atome de chlore sur l'une au moins des positions méta par rapport à l'hydroxyle phénolique par hydrodéchloration à chaud en phase liquide d'un polychlorophénol de formule générale (I):

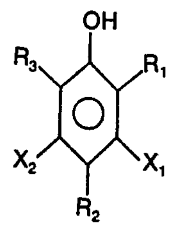

(I)

dans laquelle:

– $X_1$ et $X_2$, identiques ou différents représentent un atome de chlore, un atome d'hydrogène, un radical alkyle, aryle, arylalkyle, alkoxy, aryloxy, l'un au moins des symboles $X_1$ et $X_2$ représentant un atome de chlore,

– $R_1$, $R_2$, et $R_3$, identiques ou différents, représentent un atome de chlore, un atome d'hydrogène, un radical alkyle, un radical aryle, arylalkyle, un radical alkoxy, aryloxy, l'un au moins des symboles $R_1$, $R_2$, et $R_3$ représentant un atome de chlore,

caractérisé en ce que l'on fait réagir ledit polychlorophénol avec de l'acide iodhydrique en quantité suffisante par rapport aux atomes de chlore à éliminer du polychlorophénol (I).

Plus spécifiquement ceux des radicaux $X_1$, $X_2$, $R_1$, $R_2$, et $R_3$ qui ne symbolisent pas un atome de chlore représentent un atome d'hydrogène, un radical alkyle comportant de 1 à 10 atomes de carbone et de préférence de 1 à 4 atomes de carbone comme les radicaux méthyle, éthyle, n-propyle, isopropyle, n-butyle, isobutyle, t-butyle; un radical phényle; un radical benzyle; un radical alkoxy comportant de 1 à 10, et de préférence de 1 à 4 atomes de carbone comme les radicaux méthoxy, éthoxy, n-propoxy, isopropoxy, n-butoxy; le radical phénoxy.

La réaction de déchloration peut être représentée par le schéma:

$$\text{polychlorophénol} + 2n\ HI \rightarrow \text{métachlorophénol} + nI_2 + n\ HCl$$

n représentant le nombre (1, 2 ou 3) d'atomes de chlore à éliminer par molécule de polychlorophénol.

Le rapport molaire acide iodhydrique/nombre d'atomes de chlore du composé (I) à éliminer est généralement au moins égal à 2, afin que les réactifs soient au moins en quantités stoechiométriques. Il est bien évident que l'on peut utiliser des rapports plus faibles, mais il est économiquement plus intéressant d'avoir une réaction complète, afin de limiter les problèmes de séparation ultérieure des constituants de la masse réactionnelle finale.

Bien entendu l'acide iodhydrique peut être engagé soit sous forme gazeuse, soit sous forme de solution, notamment de solution aqueuse. Il peut également être formé in situ dans les conditions de la réaction, par exemple par action d'acide chlorhydrique sur un iodure alcalin.

Habituellement ledit rapport molaire est composé entre 2 et 20 et de préférence entre 2 et 10.

Le milieu liquide dans lequel s'effectue la réaction de l'hydrodéchloration peut être constitué soit par l'eau, soit par un solvant organique inerte dans les conditions de la réaction, notamment vis-à-vis de l'acide iodhydrique et du polychlorophénol engagés et de l'iode et de l'acide chlorhydrique formés, soit encore par un mélange d'eau et d'un tel solvant organique en toutes proportions.

Lorsqu'ils sont utilisés conjointement avec l'eau, ces solvants organiques ne sont pas impérativement miscibles à l'eau; leur rôle consiste à dissoudre les polychlorophénols mis en œuvre, car ces composés ne sont que partiellement solubles dans l'eau.

Comme exemples de ces solvants organiques on peut citer des hydrocarbures aliphatiques tels que l'octane, l'hexane; des hydrocarbures aromatiques tels que le benzène, le toluène, les xylènes; des hydrocarbures aromatiques chlorés tels que le monochlorobenzène, les dichlorobenzénes et les trichlorobenzènes.

Parmi ces solvants le monochlorobenzène, les dichlorobenzènes et les trichlorobenzènes sont particulièrement intéressants. Essentiellement en raison de leurs points d'ébullition, on utilise encore plus préférentiellement les dichlorobenzènes et les trichlorobenzènes.

Lorsque le milieu liquide de la réaction est aqueux ou partiellement aqueux, il est avantageux pour accélérer la réaction, qu'un donneur de protons tel qu'un acide minéral fort soit présent dans le milieu. Ce donneur de protons peut être un excès d'acide iodhydrique par rapport à la stoechiométrie ou un autre hydracide comme l'acide chlorhydrique, l'acide bromhydrique ou l'acide fluorhydrique ou un autre acide fort tel que l'acide sulfurique ou l'acide nitrique.

Lorsque le milieu de la réaction est uniquement organique il est nécessaire, pour que la vitesse de déchloration soit suffisante, d'introduire un acide de Lewis.

Par acide de Lewis on désigne, conformément à la définition usuelle, des composés accepteurs de doublets électroniques. Pour la mise en œuvre de la présente invention, on pourra faire appel à tous les types d'acides de Lewis et notamment à ceux cités dans l'ouvrage édité par G.A. OLAH «Friedel-Crafts and Related Reactions» 1963 tome I, pages 191 à 197. Parmi les acides de Lewis, on utilise de préférence les halogénures acides, cf.: G.A. OLAH loc. cit. pages 215 à 219 et plus particulièrement, les halogénures d'éléments des groupes 3a, 4a, 5a, 1b, 2b, 4b, 5b, 6b, 7b et 8 de la classification périodique des éléments (cf. Handbook of Chemistry and Physics édité par R.C. WEAST 53ème édition 1972–1973) tels que les chlorures, bromures, fluorures et iodures de bore, d'aluminium, d'étain, de phosphore, d'arsenic, de bismuth, de titane, de zirconium, de vanadium, de molybdène, de fer, de cobalt, de nickel, de cuivre, de zinc et de cadmium. Comme exemples spécifiques de tels halogénures on peut citer: le trichlorure d'aluminium, le tribromure d'aluminium, le triiodure d'aluminium, les chlorures stannique et stanneux, les bromures stannique et stanneux, le trichlorure de bismuth, le tribromure de bismuth, le trifluorure de bore et ses complexes avec des composés électrodonneurs tels que les éthers (par exemple le diéthyltrifluoroéthérate de bore), le trichlorure de bore, le tribromure de bore, les tétrachlorures de titane, de zirconium et de vanadium, les chlorures de molybdène, le chlorure ferrique, le bromure ferrique, le chlorure cuivreux, le chlorure cuivrique, le chlorure de zinc.

Parmi les halogénures cités précédemment, on met en œuvre de préférence le trichlorure et le tribromure d'aluminium.

Il est bien clair que l'on peut indifféremment utiliser un seul acide de Lewis ou un mélange de plusieurs acides de Lewis dans la mise en œuvre du procédé selon l'invention.

La quantité d'acide de Lewis exprimée par le rapport molaire acide de Lewis/polychlorophénol peut varier dans de larges limites. De préférence la quantité d'acide de Lewis est calculée pour que le rapport molaire précité soit d'au moins 0,01 et de préférence d'au moins 0,1. Il n'y a pas de limite supérieure critique de ce rapport, mais pour des raisons pratiques évidentes, il n'y a pas lieu qu'il soit supérieur à 2 et de préférence supérieur à 1.

La pression sans laquelle s'effectue la réaction d'hydrodéchloration est la pression autogène des différents réactifs.

Cependant lorsque le milieu liquide est constitué par un solvant organique, la pression croît au cours de la réaction, car celle-ci dégage de l'acide chlorhydrique. On préfère dans ce cas limiter la pression à au plus 100 bars et de préférence à au plus 50 bars. Pour cela on peut effectuer périodiquement un dégazage; il est bon alors de compenser les éventuelles pertes en acide iodhydrique par un apport de ce réactif. Il est également possible de faire circuler un courant d'acide iodhydrique dans le milieu réactionnel tout en éliminant en continu l'acide chlorhydrique formé. Lors de ces divers ajustements de la pression il ne faut pas abaisser celle-ci au-dessous de 3 bars et de préférence au-dessous de 5 bars.

La température de la réaction d'hydrodéchloration est généralement comprise entre 90 °C et 250 °C. A des températures plus basses la réaction est lente; des températures plus élevées ne présentent pas d'intérêt industriel.

De préférence la température à laquelle on opère est située entre 110 °C et 220 °C.

Comme exemples de polychlorophénols de formule (I) qui peuvent être utilisés comme matières premières dans le procédé conforme à la présente invention, on peut citer:

- le dichloro-2,3 phénol
- le dichloro-2,5 phénol
- le dichloro-3,4 phénol
- le trichloro-2,3,4 phénol
- le trichloro-2,3,6 phénol
- le trichloro-2,4,5 phénol
- le trichloro-2,3,5 phénol
- le trichloro-3,4,5 phénol
- le tétrachloro-2,3,4,6 phénol
- le tétrachloro-2,3,4,5 phénol
- le tétrachloro-2,3,5,6 phénol
- le pentachlorophénol
- le trichloro-2,3,4 méthyl-6 phénol
- le dichloro-2,3 méthyl-6 phénol
- le tétrachloro-2,3,4,6 méthyl-5 phénol
- le dichloro-2,3 méthyl-4 phénol
- le tétrachloro-2,3,5,6 méthyl-4 phénol
- le dichloro-2,5 diméthyl-3,4 phénol
- le dichloro-2,5 éthyl-4 phénol
- le dichloro-2,5 propyl-4 phénol
- le dichloro-2,5 t-butyl-4 phénol
- le trichloro-3,4,6 benzyl-2 phénol
- le dichloro-3,4 méthoxy-2 phénol
- le dichloro-3,6 méthoxy-2 phénol
- le dichloro-4,5 méthoxy-2 phénol
- le dichloro-5,6 méthoxy-2 phénol
- le trichloro-3,4,6 méthoxy-2 phénol
- le trichloro-3,4,5 méthoxy-2 phénol
- le tétrachloro-3,4,5,6 méthoxy-2 phénol
- le dichloro-4,5 méthoxy-3 phénol
- le dichloro-5,6 méthoxy-3 phénol
- le dichloro-2,5 méthoxy-3 phénol
- le trichloro-4,5,6 méthoxy-3 phénol
- le trichloro-2,3,6 méthoxy-3 phénol
- le dichloro-4,5 phénoxy-2 phénol
- le tétrachloro-2,3,5,6 phénoxy-4 phénol
- le dichloro-3,4 éthoxy-2 phénol
- le trichloro-3,4,5 éthoxy-2 phénol
- le dichloro-3,4 phényl-2 phénol
- le trichloro-3,5,6 phényl-2 phénol.

Parmi les phénols comportant un atome de chlore sur l'une au moins des positions en méta par rapport à l'hydroxyle phénolique qui peuvent être préparés par le procédé selon la présente invention, on peut citer:

- le chloro-3 phénol
- le dichloro-3,5 phénol
- le chloro-3 méthyl-6 phénol
- le chloro-3 méthyl-5 phénol
- le chloro-3 méthyl-4 phénol
- le dichloro-3,5 méthyl-4 phénol
- le chloro-5 diméthyl-3,4 phénol
- le dichloro-3,5 éthyl-4 phénol
- le dichloro-3,5 propyl-4 phénol
- le dichloro-3,5 t-butyl-4 phénol
- le chloro-3 benzyl-2 phénol
- le chloro-3 méthoxy-2 phénol
- le chloro-3 méthoxy-6 phénol
- le dichloro-3,5 méthoxy-2 phénol
- le chloro-3 méthoxy-5 phénol
- le chloro-3 phénoxy-6 phénol
- le dichloro-3,5 phénoxy-6 phénol
- le chloro-3 éthoxy-2 phénol
- le chloro-3 phényl-2 phénol.

Le procédé selon l'invention peut être mis en œuvre de façon continue ou discontinue. En fin de réaction le ou les phénols métachlorés peuvent être séparés du milieu réactionnel par tout moyen connu en soi, par exemple par extraction à l'aide d'un solvant et/ou par distillation.

Le procédé selon l'invention est particulièrement avantageux car il permet l'obtention sélective de phénols métachlorés en opérant sans catalyseur et sous des pressions relativement modérées.

Les exemples suivants illustrent l'invention et montrent comment elle peut être mise en pratique.

Exemple 1.

Dans un autoclave de 225 $cm^3$ de capacité, revêtu intérieurement de Tantale, on charge:
0,08 mole de pentachlorophénol
0,04 mole de chlorure d'aluminium anhydre
40 $cm^3$ d'orthodichlorobenzène.
On ferme l'autoclave, élimine l'air qu'il contient par une purge avec de l'azote, puis on le refroidit. On le met alors en communication avec une bouteille contenant de l'acide iodhydrique sec et liquéfié, de telle sorte qu'on charge dans le réacteur 0,64 mole d'HI.

La température du réacteur ainsi chargé est portée à 135 °C sous pression autogène et on laisse réagir 1 h 30 mn à cette température.

Le réacteur est ensuite refroidi, dégazé et vidé de son contenu qui est hydrolysé par 50 $cm^3$ d'une solution aqueuse contenant 1 mole d'HCl/litre.

La phase organique contenant l'iode et les polychlorophénols est séparée de la phase aqueuse. Cette dernière est débarrassée de l'iode et des chlorophénols qu'elle contient encore par un lavage avec une quantité appropriée de dichloro-1,2 benzène.

L'iode est éliminé (et ainsi est récupéré) de la phase organique par distillation avec une partie du dichlorobenzène tandis que les polychlorophénols moins volatils restent dans le bouilleur.

L'analyse par chromatographie en phase vapeur (CPV) du contenu du bouilleur indique que le taux de transformation (TT) du pentachlorophénol est de 100%. Le rendement, par rapport au pentachlorophénol engagé au départ, en dichloro-3,5 phénol est de 96,7%.

Il reste en outre du trichloro-2,3,5 phénol (RR:2,3%) et du tétrachloro-2,3,5,6 phénol (RR :1%) intermédiaires de la réaction.

Exemple 2.

On opère comme dans l'exemple 1, mais on ne charge que 0,48 mole de HI.

Après 1 heure de réaction à 135 °C on constate que le TT du pentachlorophénol (PCP) est de 94%, que le RR en dichloro-3,5 phénol (3,5 DCP) est de 60%.

En outre on trouve:

RR en tétrachloro-2,3,5,6 phénol (2,3,5,6 TTCP) = 19,5%

RR en trichloro 2,3,5 phénol (2,3,5 TCP) = 13,5%

Ces derniers peuvent éventuellement être récupérés et recyclés.

Exemple 3.

On opère comme dans l'exemple 1, mais on charge 0,02 mole d'$AlCl_3$ au lieu de 0,04 mole.

Après 15 heures de réaction à 135 °C on trouve que le taux de transformation du PCP est de 100%.

Le RR en 3,5 DCP est de 73,3%.

On a en outre: RR en 2,3,5,6 TTCP non transformé = 5%

RR en 2,3,5 TCP non transformé = 0,7%

RR en 3 chlorophénol (3 CP) = 19%

Exemple 4.

On opère comme dans l'exemple 1, mais on charge 0,008 mole d'$AlCl_3$ au lieu de 0,04 mole.

Après 8 heures de réaction à 150 °C on trouve que le taux de transformation du PCP est de 100%.

RR en 2,3,5,6 TTCP = 6,9%

RR en 2,3,5 TCP non transformé = 3,5%

RR en 3,5 DCP = 77%.

Il s'est en outre formé du 3 CP : RR = 12%

Exemple 5.

On opère comme dans l'exemple 1, mais on remplace le PCP par 2,3,4,6 TTCP.

Après 6 heures de réaction à 135 °C on trouve que le taux de transformation du 2,3,4,6 TTCP est de 97%.

RR en 3 CP = 77%.

Il reste en outre: du 2,3,6 TCP (RR = 4%)
du dichlorophénol (2,3 et 2,5 DCP) (RR = 9,5%)

Il s'est formé également: du phénol (RR = 4,9%)
du chloro-2 phénol (RR: 1%)

Exemple 6.

On répète l'exemple 1, en utilisant le 2,3,6 TCP comme substrat, avec les charges suivantes:

2,3,6 TCP: 0,08 mole
HI: 0,427 mole
$AlCl_3$: 0,04 mole

Après 6 heures de réaction à 135 °C on trouve que le taux de transformation du 2,3,6 TCP est de 100%.

RR en 3 CP = 86%.

Il reste en outre: du 2,3 DCP (RR = 6%)
du dichloro-2,6 phénol (RR = 4%)

Il s'est formé également: du phénol (RR = 3,7%)

Exemple 7. (milieu aqueux)

Dans un autoclave de 225 $cm^3$ de capacité, revêtu intérieurement de Tantale, on charge:

0,05 mole de 3,4 DCP

100 $cm^3$ d'une solution aqueuse d'HI à 4 moles/par litre

On ferme l'autoclave, élimine l'air qu'il contient par une purge avec de l'azote. On porte la température de l'autoclave à 190 °C sous pression autogène et on maintient ces conditions pendant 2 heures.

La masse réactionnelle est extraite par du toluène.

Après élimination de l'iode l'analyse par CPV donne:

TT du 3,4 DCP = 98%
RR en 3 CP = 85,3%
RR en 4 CP = 12,6%

Exemples 8 à 12

Dans les exemples 8 à 12 on répète l'opération décrite dans l'exemple 7, mais on fait varier soit le substrat, soit la quantité de HI, sa concentration dans l'eau et éventuellement la concentration initiale en protons par ajout d'acide chlorhydrique.

Les résultats obtenus sont rassemblés dans le tableau ci-après:

Tableau 1

| Exemples | Substrat (m. moles) | HI (m. moles) | HCl (m. moles) | $H_2O$ (cm3) | Temps (°C) | Durée (h.) | TT % | RR % 3 CP | RR % 4 CP | RR % 2 CP |
|---|---|---|---|---|---|---|---|---|---|---|
| 8 | 3,4 DCP 50 | 100 | 0 | 25 | 190 | 2 | 62 | 59,7 | 2,7 | 0 |
| 9 | 3,4 DCP 50 | 100 | 0 | 50 | 190 | 4 | 54 | 54 | 0 | 0 |
| 10 | 3,4 DCP 50 | 100 | 900 | 100 | 190 | 2 | 79 | 71,5 | 7,8 | 0 |
| 11 | 2,3 DCP 50 | 400 | 0 | 100 | 190 | 2 | 75 | 75 | 0 | 0 |
| 12 | 2,3 DCP 50 | 100 | 0 | 25 | 190 | 4 | 85 | 85 | 0 | 0 |

Exemple 13

On répète l'exemple 7 en chargeant:

| | | |
|---|---|---|
| 2,3,6 TCP | = | 25 m.moles |
| HI | = | 120 m.moles |
| HCl | = | 840 m.moles |
| Eau | = | 100 cm$^3$ |

Après 16 heures de réaction à 190 °C sous pression autogène, on a les résultats suivants:

| | | |
|---|---|---|
| TT du 2,3,6 TCP | = | 100% |
| RR en 3 CP | = | 72% |
| RR en 2,3 DCP | = | 10% |
| RR en 2 CP | = | 8% |
| RR en 2,6 DCP | = | 10% |

Exemple 14

On répète l'exemple 7 en chargeant:

| | | |
|---|---|---|
| PCP | = | 16 m.moles |
| HI | = | 160 m.moles |
| HCl | = | 960 m.moles |
| Eau | = | 100 cm$^3$ |

Après 6 heures de réaction à 190 °C sous pression autogène, on a les résultats suivants:

| | | |
|---|---|---|
| TT du PCP | = | 92% |
| RR en 3,5 DCP | = | 76,3% |
| RR en 2,3,5 TCP | = | 11,3% |
| RR en 2,3,5,6 TTCP | = | 3,1% |

Exemple 15

On répète l'exemple 7 en chargeant:

| | | |
|---|---|---|
| PCP | = | 10 m.moles |
| HI | = | 80 m.moles |
| Orthodichlorobenzène | = | 15 cm$^3$ |
| Eau | = | 10 cm$^3$ |

Après 16 heures de réaction à 190 °C sous pression autogène, on a les résultats suivants:

| | | |
|---|---|---|
| TT du PCP | = | 100% |
| RR en 3,5 DCP | = | 78% |
| RR en 2,3,5 TCP | = | 13,3% |
| RR en 2,3,5,6 TTCP | = | 8,7% |

**Revendications**

1. Procédé de préparation sélective de chlorophénol comportant un atome de chlore sur l'une au moins des positions méta par rapport à l'hydroxyle phénolique, par hydrodéchloration à chaud en phase liquide, d'un polychlorophénol de formule générale (I):

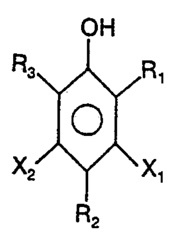

(I)

dans laquelle:

– $X_1$ et $X_2$, identiques ou différents représentent un atome de chlore, un atome d'hydrogène, un radical alkyle, aryle, arylalkyle, alkoxy, aryloxy, l'un au moins des symboles $X_1$ et $X_2$ représentant un atome de chlore,

– $R_1$, $R_2$, et $R_3$, identiques ou différents, représentent un atome de chlore, un atome d'hydrogène, un radical alkyle, un radical aryle, arylalkyle, un radical alkoxy, aryloxy, l'un au moins des symboles $R_1$, $R_2$, et $R_3$ représentant un atome de chlore, caractérisé en ce que l'on fait réagir ledit polychlorophénol (I) avec de l'acide iodhydrique en quantité suffisante par rapport aux atomes de chlore à éliminer du polychlorophénol (I).

2. Procédé selon la revendication 1, caractérisé en ce que le rapport molaire acide iodhydrique/nombre d'atomes de chlore à éliminer par molécule de polychlorophénol (I) est compris entre 2 et 20.

3. Procédé selon la revendication 1, caractérisé en ce que le rapport molaire acide iodhydrique/nombre d'atomes de chlore à éliminer par molécule de polychlorophénol (I) est compris entre 2 et 10.

4. Procédé selon l'une des revendications 1 à 3, caractérisé en ce que ceux des radicaux $X_1$, $X_2$, $R_1$, $R_2$ et $R_3$ qui ne symbolisent pas un atome de chlore représentent un atome d'hydrogène, un radical alkyle comportant de 1 à 10 atomes de carbone; un radical phényle; un radical benzyle; un radical alkoxy comportant de 1 à 10 atomes de carbone; un radical phénoxy.

5. Procédé selon l'une des revendications 1 à 4, caractérisé en ce que le milieu de la réaction est constitué par de l'eau ou par un mélange eau/solvant organique inerte.

6. Procédé selon la revendication 5, caractérisé en ce que la réaction est effectuée en présence d'un acide minéral fort.

7. Procédé selon la revendication 6, caractérisé en ce que l'acide minéral fort est l'acide chlorhydrique.

8. Procédé selon l'une des revendications 1 à 4, caractérisé en ce que le milieu de la réaction est constitué par un solvant organique inerte et que ladite réaction est conduite en présence d'un acide de Lewis.

9. Procédé selon la revendication 8, caractérisé en ce que l'acide de Lewis utilisé est un halogénure d'éléments des groupes 3a, 4a, 5a, 1b, 2b, 4b, 5b, 6b, 7b et 8 de la classification périodique des éléments.

10. Procédé selon l'une des revendications 8 et 9, caractérisé en ce que l'acide de Lewis est un halogénure d'aluminium et de préférence le chlorure ou le bromure d'aluminium.

11. Procédé selon l'une des revendications 8 à 10, caractérisé en ce que la quantité d'acide de Lewis est telle que le rapport molaire acide de Lewis/polychlorophénol de formule (I) est d'au moins 0,01 et de préférence d'au moins 0,1.

12. Procédé selon l'une des revendications 8 à 11, caractérisé en ce que la quantité d'acide de Lewis est telle que le rapport molaire acide de Lewis/polychlorophénol de formule (I) est au plus de 2 et de préférence au plus de 1.

13. Procédé selon l'une des revendications 1 à 12, caractérisé en ce que la température est comprise entre 90 °C et 250 °C.

14. Procédé selon l'une des revendications 1 à 13, caractérisé en ce que la température est comprise entre 110 °C et 220 °C.

15. Procédé selon l'une des revendications 1 à 14, caractérisé en ce que la pression autogène est au plus égale à 100 bars et de préférence au plus égale à 50 bars.

16. Procédé selon l'une des revendications 8 à 12, caractérisé en ce que la pression est maintenue au moins égale à 3 bars.

17. Procédé selon l'une des revendications 1 à 16, caractérisé en ce qu'il est appliqué à la déchloration du pentachlorophénol, du tétrachloro-2,3,4,6 phénol, du trichloro-2,3,6 phénol ou du dichloro-3,4 phénol.

**Claims**

1. Process for the selective preparation of chlorophenol containing one chlorine atom on at least one of the meta-positions relative to the phenolic hydroxyl, by liquid-phase hot hydrodechlorination of a polychlorophenol of the general formula (I):

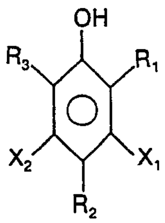

(I)

in which:

$X_1$ and $X_2$, which may be identical or different, represent a chlorine atom, a hydrogen atom or an alkyl, aryl, arylalkyl, alkoxy or aryloxy radical, with at least one of the symbols $X_1$ and $X_2$ representing a chlorine atom,

$R_1$, $R_2$ and $R_3$, which may be identical or different, represent a chlorine atom, a hydrogen atom, an alkyl radical, an aryl or arylalkyl radical or an alkoxy or aryloxy radical, with at least one of the symbols $R_1$, $R_2$ and $R_3$ representing a chlorine atom, characterized in that the said polychlorophenol (I) is reacted with hydriodic acid in a sufficient amount, relative to the chlorine atoms to be removed from the polychlorophenol (I).

2. Process according to Claim 1, characterized in that the molar ratio of hydriodic acid/number of chlorine atoms to be removed per molecule of polychlorophenol (I) is between 2 and 20.

3. Process according to Claim 1, characterized in that the molar ratio of hydriodic acid/number of chlorine atoms to be removed per molecule of polychlorophenol (I) is between 2 and 10.

4. Process according to one of Claims 1 to 3, characterized in that those of the radicals $X_1$, $X_2$, $R_1$, $R_2$ and $R_3$ which do not symbolize a chlorine atom represent a hydrogen atom, an alkyl radical containing from 1 to 10 carbon atoms, a phenyl radical, a benzyl radical, an alkoxy radical containing from 1 to 10 carbon atoms or a phenoxy radical.

5. Process according to one of Claims 1 to 4, characterized in that the reaction medium consists of water or of a mixture of water and an inert organic solvent.

6. Process according to Claim 5, characterized in that the reaction is carried out in the presence of a strong mineral acid.

7. Process according to Claim 6, characterized in that the strong mineral acid is hydrochloric acid.

8. Process according to one of Claims 1 to 4, characterized in that the reaction medium consists of an inert organic solvent and that the said reaction is carried out in the presence of a Lewis acid.

9. Process according to Claim 8, characterized in that the Lewis acid used is a halide of elements of groups 3a, 4a, 5a, 1b, 2b, 4b, 5b, 6b, 7b and 8 of the periodic classification of the elements.

10. Process according to one of Claims 8 and 9, characterized in that the Lewis acid is an aluminium halide and preferably aluminium chloride or aluminium bromide.

11. Process according to one of Claims 8 to 10, characterized in that the amount of Lewis acid is such that the molar ratio of Lewis acid/polychlorophenol of formula (I) is at least 0.01 and preferably at least 0.1.

12. Process according to one of Claims 8 to 11, characterized in that the amount of Lewis acid is such that the molar ratio of Lewis acid/polychlorophenol of formula (I) is at most 2 and preferably at most 1.

13. Process according to one of Claims 1 to 12, characterized in that the temperature is between 90 °C and 250 °C.

14. Process according to one of Claims 1 to 13, characterized in that the temperature is between 110 °C and 220 °C.

15. Process according to one of Claims 1 to 14, characterized in that the autogenous pressure is at most equal to 100 bars and preferably at most equal to 50 bars.

16. Process according to one of Claims 8 to 12, characterized in that the pressure is kept at least equal to 3 bars.

17. Process according to one of Claims 1 to 16, characterized in that it is applied to the dechlorination of pentachlorophenol, of 2,3,4,6-tetrachlorophenol, of 2,3,6-trichlorophenol or of 3,4-dichlorophenol

**Patentansprüche**

1. Verfahren zur selektiven Herstellung von Chlorphenol, das ein Chloratom an mindestens einer der meta-Stellungen in bezug auf das phenolische Hydroxyl trägt, durch Enthydrochlorierung in der Wärme in flüssiger Phase eines Polychlorphenols der allgemeinen Formel (I)

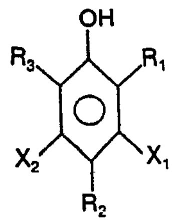

(I)

worin:

$X_1$ und $X_2$, die identisch oder verschieden sind, ein Chloratom, ein Wasserstoffatom, einen Alkyl-, Aryl-, Arylalkyl-, Alkoxy-, Aryloxyrest bedeuten, wobei wenigstens eines der Symbole $X_1$ und $X_2$ ein Chloratom darstellt,

$R_1$, $R_2$ und $R_3$, die identisch oder verschieden sind, ein Chloratom, ein Wasserstoffatom, einen Alkylrest, einen Aryl-, Arylalkylrest, einen Alkoxy-, Aryloxyrest bedeuten, wobei wenigstens eines der Symbole $R_1$, $R_2$ und $R_3$ ein Chloratom darstellt, dadurch gekennzeichnet, dass man dieses Polychlorphenol (I) mit Jodwasserstoffsäure in einer Menge, die in bezug auf die zu entfernenden Chloratome des Polychlorphenols (I) ausreichend ist, zur Reaktion bringt.

2. Verfahren gemäss Anspruch 1, dadurch gekennzeichnet, dass das Molverhältnis Jodwasserstoffsäure/Anzahl zu entfernender Chloratome pro Mol Polychlorphenol (I) zwischen 2 und 20 beträgt.

3. Verfahren gemäss Anspruch 1, dadurch gekennzeichnet, dass das Molverhältnis Jodwasserstoffsäure/Anzahl zu entfernender Chloratome pro Mol Polychlorphenol (I) zwischen 2 und 10 beträgt.

4. Verfahren gemäss einem der Ansprüche 1 bis 3, dadurch gekennzeichnet, dass diejenigen der Reste $X_1$, $X_2$, $R_1$, $R_2$ und $R_3$, die kein Chloratom bedeuten, ein Wasserstoffatom, einen Alkylrest mit 1 bis 10 Kohlenstoffatomen, einen Phenylrest, einen Benzylrest, einen Alkoxyrest mit 1 bis 10 Kohlenstoffatomen, einen Phenoxyrest darstellen.

5. Verfahren gemäss einem der Ansprüche 1 bis 4, dadurch gekennzeichnet, dass das Reaktionsmilieu aus Wasser oder einem Gemisch Wasser/inertes, organisches Lösungsmittel besteht.

6. Verfahren gemäss Anspruch 5, dadurch gekennzeichnet, dass die Reaktion in Anwesenheit einer starken Mineralsäure bewirkt wird.

7. Verfahren gemäss Anspruch 6, dadurch gekennzeichnet, dass die starke Mineralsäure Chlorwasserstoffsäure ist.

8. Verfahren gemäss einem der Ansprüche 1 bis 4, dadurch gekennzeichnet, dass das Reaktionsmilieu aus einem inerten, organischen Lösungsmittel besteht und dass diese Reaktion in Anwesenheit einer Lewissäure durchgeführt wird.

9. Verfahren gemäss Anspruch 8, dadurch gekennzeichnet, dass die verwendete Lewissäure ein Halogenid der Elemente der Gruppen 3a, 4a, 5a, 1b, 2b, 4b, 5b, 6b, 7b und 8 des Periodensystems der Elemente ist.

10. Verfahren gemäss einem der Ansprüche 8 und 9, dadurch gekennzeichnet, dass die Lewissäure ein Aluminiumhalogenid und vorzugsweise Aluminiumchlorid oder -bromid ist.

11. Verfahren gemäss einem der Ansprüche 8 bis 10, dadurch gekennzeichnet, dass die Menge an Lewissäure derart ist, dass das Molverhältnis Lewissäure/Polychlorphenol der Formel (I) mindestens 0,01 und vorzugsweise mindestens 0,1 beträgt.

12. Verfahren gemäss einem der Ansprüche 8 bis 11, dadurch gekennzeichnet, dass die Menge an Lewissäure derart ist, dass das Molverhältnis Lewissäure/Polychlorphenol der Formel (I) höchstens 2 und vorzugsweise höchstens 1 beträgt.

13. Verfahren gemäss einem der Ansprüche 1 bis 12, dadurch gekennzeichnet, dass die Temperatur zwischen 90 °C und 250 °C liegt.

14. Verfahren gemäss einem der Ansprüche 1 bis 13, dadurch gekennzeichnet, dass die Temperatur zwischen 110 °C und 220 °C liegt.

15. Verfahren gemäss einem der Ansprüche 1 bis 14, dadurch gekennzeichnet, dass der autogene Druck höchstens gleich 100 bar und vorzugsweise höchstens gleich 50 bar ist.

16. Verfahren gemäss einem der Ansprüche 8 bis 12, dadurch gekennzeichnet, dass der Druck bei mindestens gleich 3 bar gehalten wird.

17. Verfahren gemäss einem der Ansprüche 1 bis 16, dadurch gekennzeichnet, dass Pentachlorphenol, 2,3,4,6-Tetrachlorphenol, 2,3,6-Trichlorphenol oder 3,4-Dichlorphenol zur Entchlorierung angewandt wird.